# EUROPEAN PATENT APPLICATION

(11) **EP 0 947 581 A1**
(43) Date of publication of application: **06.10.1999**
(21) Application number: 97934720.0
(22) Date of filing: 06.08.1997
(51) Int. Cl.: C12N 5/02, C12N 5/06, C12N 5/08, C12N 5/10, C12N 5/20, C12N 5/24, C12P 21/00, C12P 21/02, C12P 21/08

(54) **CULTURE MEDIUM AND USE OF THE SAME**

(30) Priority: 08.08.1996 JP 21000496
(71) Applicant: YOSHITOMI PHARMACEUTICAL INDUSTRIES, LTD., Osaka-shi Osaka 541-0046 (JP)
(72) Inventor: AMATSUJI, Yasuo, Yoshitomo Pharmaceutic.Ind., Ltd., Hirakata-shi, Osaka 573-1153 (JP); TAMASHIMA, Hiroshi, Yoshitomi Pharmaceut.Ind., Ltd, Hirakata-shi, Osaka 573-1153 (JP); KOBAYASHI, Kaoru, Yoshitomi Pharmaceutic.Ind., Ltd, Hirakata-shi, Osaka 573-1153 (JP); OHMURA, Takao, Yoshitomo Pharmaceutical Ind.,Ltd., Osaka-shi, Osaka 536-0005 (JP)
(74) Representative: VOSSIUS & PARTNER
(86) International application number: JP9702749
(87) International publication number: WO9806822

(57) **Abstract**

The present invention relates to a medium for culture, containing human serum albumin (rHSA) obtained by gene manipulation, a method for culturing an animal cell, including culturing the animal cell in the above-mentioned medium for culture, and a method for producing a physiologically active substance, including culturing an animal cell capable of producing a physiologically active substance in the above-mentioned medium for culture to produce the physiologically active substance and harvesting the physiologically active substance from the culture. The use of rHSA having constant quality has led to the effects of retention of cell proliferation capability and production of physiologically active substance, which are of the same level as achieved when a serum-containing medium and/or a conventional serum-free medium containing plasma-derived HSA are/is used, as well as stable quality of the medium and ensured reproducibility of the culture.

## Description

### Technical Field

The present invention relates to a medium for culture, which contains human serum albumin obtained by gene manipulation (hereinafter also referred to as rHSA), a method for culturing animal cells using said medium and a method for producing and harvesting a physiologically active substance.

### Background Art

With the progress of DNA recombination technique in recent years, methods for producing a physiologically active substance, such as protein, as a cell product have been investigated. While culture of animal cells is critical for the production of such cell product, cell culture in an industrial scale requires a great amount of a medium.

There have been conventionally used a great variety of media for growing (proliferating) animal cells, which include Dulbecco's modified Eagle's medium (DMEM) [Morton, H, J. J. (1970) In vitro 6, 89], F12 medium [Ham, R. O. (1965) Proc. Natl. Acad. Sci. USA 53, 288], RPMI1640 medium [Goding, J. W. (1980) J. Immunol. Methods 39, 285, JAMA 199 (1957) 519] and the like. These media require addition of serum to the medium for the growth (proliferation) of animal cells. Typically, fetal bovine serum, equine serum, human serum and the like need to be added in a concentration of about 1-15%.

The use of a medium containing a serum is associated with the following problems.
(1) Serum itself is expensive and the culture becomes costly.
(2) Serum varies between lots, which is disadvantageous for culture requiring reproducibility.
(3) Purification from the culture supernatant of a cell product is difficult.
(4) The medium is associated with a risk of providing a contamination source of virus and mycoplasma.

Under the circumstances, a method for decreasing the serum concentration of a medium has been investigated. However, decrease in the serum concentration leads to significant degradation of proliferation capability of the cell or eradication of the cell, which in turn results in a noticeable decrease in the yield of a desired cell product (e.g., physiologically active substance such as protein). Hence, decreasing concentration of the serum in a medium has been unfeasible.

In view of the above, a serum-free medium wherein cells can be cultured without losing proliferation capability in the absence of serum has been drawing much attention.

Conventional serum-free media contain a plasma-derived human serum albumin (hereinafter also referred to as HSA) as an additive for maintaining proliferation capability of the cell. The plasma-derived HSA does not necessarily has constant quality. For example, the level of contaminant plasma-derived components, such as lipoprotein, protease inhibitor (e.g., α1-antitrypsin and the like), carrier protein of metal or heme (e.g., transferrin, ceruloplasmin, haptoglobin and the like), fatty acid, calcium ion, tryptophan, cysteine, glutathione, trace amount of metal component and the like, varies from one product lot to another. Thus, there is a concern that these components may expert influence on the culture of animal cells and the like.

### Disclosure of the Invention

The present invention has been made in view of the above-mentioned problems, and aims at providing a medium for culture, which has stable quality, exerts less influence on the culture, and in which cells can proliferate sufficiently.

Another object of the present invention is to provide a method for providing stable (having superior reproducibility) culture of animal cells.

A yet another object the present invention is to provide a method for producing a physiologically active substance in large amounts from animal cells capable of producing the physiologically active substance and harvesting the same.

The present inventors have conducted intensive studies in consideration of the above-mentioned situation and found that the use of an HSA (rHSA) obtained by gene manipulation as an additive for the basal medium leads to retention of sufficient proliferation capability of the cell while maintaining the effect during culture as achieved by the use of a plasma-derived HSA, which resulted in the completion of the present invention. In other words, the use of rHSA having constant quality for the medium of the present invention beneficially results in less influence (i.e., ensuring reproducibility) on the effect of culture.

Accordingly, the present invention provides the following.
(1) A medium for culture, containing a human serum albumin obtained by gene manipulation.
(2) The medium for culture of the above-mentioned (1), containing, as a basal medium, DMEM medium, F12 medium or RPMI1640 medium.
(3) The medium for culture of the above-mentioned (1), wherein the human serum albumin obtained by gene manipulation is the albumin produced by a human serum albumin-producing yeast.
(4) The medium for culture of the above-mentioned (1), further containing insulin.
(5) The medium for culture of the above-mentioned (1), further containing peptone.
(6) The medium for culture of the above-mentioned (1), further containing transferrin.
(7) The medium for culture of the above-mentioned (1), further containing insulin, peptone and transferrin.
(8) A method for culturing an animal cell, comprising culturing the animal cell in the medium of the above-mentioned (1).
(9) The method of the above-mentioned (8), wherein the animal cell itself is capable of producing a physiologically active substance or producing a heterologous physiologically active substance by transformation by genetic engineering.
(10) The method of the above-mentioned (8), wherein the animal cell is a member selected from the group consisting of human kidney cell line, hybridoma, leukocyte, Namalwa cell, BALL-1 cell, fibroblast, lymphocyte, human kidney cell, melanoma cell, Vero cell, Hela cell, CHO cell, WI 38 cell, BHK cell, COS-7 cell, MDCK cell, C127 cell, HKG cell, mouse myeloma cell, human lymphoblast cell, a parent cell for preparing human-human hybridoma, dhfr deficient cell lines thereof, HGPRT deficient cell lines thereof and ouabain resistant cell lines thereof.
(11) A method for producing a physiologically active substance, comprising culturing an animal cell capable of producing the physiologically active substance in the medium of the above-mentioned (1) to produce the physiologically active substance, and harvesting the physiologically active substance from the culture.
(12) The method of the above-mentioned (11), wherein the animal cell itself is capable of producing a physiologically active substance or producing a heterologous physiologically active substance by transformation by genetic engineering.
(13) The method of the above-mentioned (11), wherein the physiologically active substance is a member selected from the group consisting of prourokinase, urokinase, antithrombin-III, tissue plasminogen activator, hepatitis B virus surface antigen, hepatitis pre S-B virus surface antigen, interferon, colony formation stimulating factor and monoclonal antibody.

The medium for culture of the present invention is subject to no particular limitation as long as it contains a human serum albumin (rHSA) obtained by gene manipulation. Specific examples include a basal medium supplemented with rHSA.

In the present invention, the basal medium is a basic medium which is typically used and contains a carbon source generally assimilable for animal cells, and nitrogen source, inorganic salt and the like that are digestable for animal cells. Where necessary, a slight amount of effective substance may be added, such as micronutrient, precursor substance and the like. The basic medium may be any known medium for cell culture, such as the above-mentioned DMEM medium, F12 medium and RPMI1640 medium, which is particularly preferably RPMI1640 medium.

The rHSA to be contained in the medium of the present invention is subject to no particular limitation as long as it is a human serum albumin produced by an HSA-producing host prepared by gene manipulation. Preferred is one substantially free of contaminant components (e.g., protein) derived from a production host, more preferably one obtained by culturing an rHSA-producing host by a known means, and harvesting and purifying from culture filtrate, microorganism or cell by known separation means and purification means.

Specific examples include the following method.

The host for obtaining the rHSA to be used in the present invention is subject to no particular limitation as long as it is prepared by gene manipulation. It may be disclosed in known publications or one to be developed from now. Examples thereof include microorganisms made rHSA-productive by gene manipulation (e.g., *E. coli*, yeast, *B. subtilis* and the like), animal cell, and the like. Particularly, the host is a yeast, preferably the genus *Saccharomyces* (e.g., *Saccharomyces cerevisiae*) or the genus *Pichia* (e.g., *Pichia pastoris*). An auxotrophic strain or antibiotic sensitive strain may be used. More preferably, *Saccharomyces cerevisiae* AH 22 strain (a, his 4, leu 2, can 1) or *Pichia pastoris* GTS 115 strain (his 4) is used.

A method for preparing these rHSA-producing hosts, a method for producing rHSA by culturing the host and a method for separating and harvesting the rHSA from culture may be known or analogous to a known method. For example, an rHSA-producing host can be prepared by using a typical HSA gene [Japanese Patent Unexamined Publication Nos. 58-56684 (corresponding to EP-A-73646), 58-90515 (corresponding to EP-A-79739), 58-150517 (corresponding to EP-A-91527)], by using a novel HSA gene [Japanese Patent Unexamined Publication No. 62-29985, Japanese Patent Unexamined Publication No. 1-98486 (both corresponding to EP-A-206733)], by using a synthetic signal sequence [Japanese Patent Unexamined Publication No. 1-240191 (corresponding to US Patent No. 5409815 and EP-A-329127)], by using a serum albumin signal sequence [Japanese Patent Unexamined Publication No. 2-167095 (corresponding to EP-A-319641)], by integrating a recombinant plasmid on a chromosome [Japanese Patent Unexamined Publication No. 3-72889 (corresponding to EP-A-399455)], by fusing hosts [Japanese Patent Unexamined Publication No. 3-53877 (corresponding to EP-A-409156)], by causing mutation in a methanol-containing medium, by using a mutant AOX₂ promoter [Japanese Patent Unexamined Publication Nos. 6-90768, 4-299984 (both corresponding to EP-A-506040)], by expressing HSA by *B. subtilis* [Japanese Patent Unexamined Publication No. 62-25133 (corresponding to EP-A-229712)], by expressing HSA by yeast [Japanese Patent Unexamined Publication Nos. 60-41487 (corresponding to EP-A-123544), 63-39576 (corresponding to EP-A-248657), 63-74493 (corresponding to EP-A-251744)], or by expressing HSA by *Pichia* yeast [Japanese Patent Unexamined Publication No. 2-104290 (corresponding to EP-A-344459)].

Of these, the method causing mutation in a methanol-containing medium includes the following steps. That is, a plasmid having a transcription unit which expresses HSA under the control of an AOX₁ promoter is introduced into the AOX₁ gene region of a suitable host by a conventional method, preferably *Pichia* yeast, specifically GTS 115 strain (NRRL deposit No. Y-15851), to give a transformant [see Japanese Patent Unexamined Publication No. 2-104290 (corresponding to EP-A-344459)]. This transformant has weak proliferation capability in a methanol medium. Therefore, this transformant is cultured in a methanol-containing medium to cause mutation and only proliferable cells are recovered. The methanol concentration here is, for example, about 0.0001-5%. The medium may be an artificial medium or natural medium. The culture conditions are 15-40°C, about 1-1000 hours.

The rHSA production host is cultured by a method disclosed in the above-mentioned publications, a method wherein high concentration cells and product are obtained by fed batch culture (semi-batch culture) by supplying high concentration glucose or methanol in suitable small amounts while avoiding high concentration substrate inhibition of production cells (Japanese Patent Unexamined Publication No. 3-83595), a method wherein a fatty acid is added to the medium to enhance rHSA production [Japanese Patent Unexamined Publication No. 4-293495 (corresponding to US Patent No. 5334512 and EP-A-504823)] and the like.

The rHSA produced by culture treatment is isolated and purified at sufficient level from the components derived from the host cell and culture components by various methods. For example, a conventional method includes subjecting a yeast culture solution containing rHSA to compression→ ultrafiltration membrane treatment→heat treatment→ultrafiltration membrane treatment, and further subjecting to column chromatography treatment with cation exchanger, hydrophobic chromatography treatment, column chromatography treatment with anion exchanger and the like [Japanese Patent Unexamined Publication No. 5-317079 (corresponding to US Patent No. 5440018 and EP-A-570916), Biotechnology of Blood Proteins. 1993, vol. 227, 293-298]. A method including, subsequent to the above-mentioned conventional method, a step of chelate resin treatment or a treatment of boric acid or salt thereof has been also documented [Japanese Patent Unexamined Publication Nos. 6-56883 and 6-245789 (both corresponding to US Patent No. 5521287 and EP-A-612761)].

Subsequent to heat treatment of this yeast culture solution, a stream line method using an adsorption fluidized bed technique [Japanese Patent Unexamined Publication No. 8-116985 (corresponding to EP-A-699687)] and the like can be also applied. The rHSA thus prepared and purified can be formulated by a known method such as sterilization by heating, ultrafiltration membrane treatment, addition of stabilizer, sterilization by filtration, dispensing, lyophilization and the like.

The rHSA is added to the medium for culture of the present invention in a proportion of about 0.1-5 g/L, preferably 0.1-2 g/L.

The medium for culture of the present invention may contain insulin, peptone, transferrin and the like as necessary.

Insulin is not particularly limited as to its derivation, but a bovine-derived insulin or a recombinant human insulin is preferably used. Peptone is not particularly limited as to its derivation, but beef-derived peptone is preferably used. A plant-derived peptone can be also used. Transferrin is not particularly limited as to its derivation, but human or bovine-derived one is preferably used.

Insulin is added to the medium for culture of the present invention in a proportion of about 0.1-10 mg/L, preferably 0.1-2 mg/L. The peptone content is 0.1-50 g/L, preferably 1-10 g/L, and the transferrin content is 0.5-20 mg/L, preferably 1-15 mg/L.

The medium for culture of the present invention may contain hypoxanthine (0.1-100 mg/L), preferably 1-20 mg/L, thymidine (0.01-100 mg/L), preferably 1-10 mg/L, selenium (0.01-100 µg/L), preferably 1-10 µg/L, and α-tocopherol (vitamin E, 0.001-10 mg/L), preferably 0.01-0.5 mg/L, where necessary.

When animal cell transformed to have a vector containing a resistance gene is to be cultured, a selection agent corresponding to the resistance gene contained in the vector may be further added to the medium to keep plasmid transformation stable. The selection agent may be those known to those of ordinary skill in the art, such as neomycin, hygromycin, micophenolic acid, hypoxanthine, xanthine, aminopterin, methotrexate (MTX) and derivatives thereof.

The animal cell that can be cultured in the medium for culture of the present invention may be capable of producing physiologically active substance by itself, or may produce a heterologous physiologically active substance by transformation by genetic engineering. The cell capable of producing physiologically active substance by itself may be, for example, hybridoma that produces monoclonal antibody, leukocyte, Namalwa cell or BALL-1 cell that produces interferon (IFN)-α, fibroblast that produces IFN-β, lymphocyte that produces IFN-γ, human kidney cell that produces prourokinase or urokinase (proUK or UK), melanoma (e.g., Bowes cell) that produces tissue plasminogen activator (TPA) and the like. The established host cell to be transformed by genetic engineering may be, for example, Vero cell, Hela cell, CHO cell, WI38 cell, BHK cell, COS-7 cell, MDCK cell, C127 cell, HKG cell, human kidney cell line and the like. Specific examples include CHO-K₁ (Chinese hamster ovary cell : ATCC CCL61), BHK (hamster kidney cell : ATCC CCL10), COS-7 (CV-1 Origin, SV-40 cell : ATCC CRL1651), Vero cell (African green monkey kidney cell : ATCC CCL81), mouse myeloma cell (X63-Ag8-653 ; P3U1), human lymphoblast cell (IM-9, ATCC CCL159), parent cell for preparing human-human hybridoma, dhfr deficient cell lines thereof, HGPRT deficient cell lines thereof, ouabain resistant cell lines thereof and the like.

An animal cell is cultured using the medium for culture of the present invention by the following method. That is, an animal cell is cultured in the inventive medium placed in a culture container, such as dish, flask, roller bottle, spinner flask, microcarrier, microcapsule and a culture apparatus using hollow fiber, which are known well for use in culture, but the container is not limited to these exemplified. The method of culture includes, for example, subculture generally performed in the above-mentioned culture container, and continuous culture performed for a long time under constant culture conditions by supplying a new culture solution complementing the old culture solution which is continuously or discontinuously extracted from the culture tank together with the cell or upon separation of the cell. Moreover, the animal cell can be cultured in the inventive medium in the form of a highly dense culture. Specifically, culture at high density of not less than 10⁷ cells/ml is preferable. Other culture conditions are, for example, culture temperature 30-37°C, culture time 1-10 days. In addition, by appropriately changing the medium, a prolonged continuous culture can be achieved.

A physiologically active substance such as protein is produced by the cell by a method known in the field of art. Examples of the method include those disclosed in Japanese Patent Unexamined Publication No. 61-177987 (corresponding to EP-A-154272), Japanese Patent Unexamined Publication No. 63-146789 (corresponding to US Patent No. 5098840 and EP-A-253241) and the like.

According to the production method of a physiologically active substance of the present invention, for example, a physiologically active substance such as antithrombin-III, proUK, UK, TPA, hepatitis B virus surface antigen, hepatitis pre S-B virus surface antigen, IFN, colony formation stimulating factor, monoclonal antibody and the like can be produced.

### Examples

The present invention is explained in more detail in the following by way of Examples which do not limit the present invention in any way.

### Example 1 Preparation of medium for culture

As a basal medium, RPMI1640 medium [10.2 g, Goding, J. W (1980) J. Immunol. Methods 39, 285, JAMA 199 (1957)] was used. As additives, rHSA (1 g), insulin (1 mg), beef-derived peptone (5 g), transferrin (10 mg), hypoxanthine (13 mg), thymidine (4 mg), α-tocopherol (0.13 mg) and selenium (4 µg) were used to give 1L of a medium for culture. The rHSA was derived from *Pichia pastoris* yeast, which was prepared according to Japanese Patent Unexamined Publication No. 8-116985.

### Example 2 Culture of human kidney cell line

Human kidney cell line was adhered to microcarrier beads and added to the medium for culture prepared in Example 1 to a concentration of 10⁷ cells/ml. The cells were cultured under the conditions of 37°C, 5% CO₂. As a result, production of proUK corresponding to 0.6-2.5 U/ml was confirmed in the culture solution in 2 days. The culture solution was changed every 2-3 days thereafter, whereby continuous culture over a long period of time (at least one month) could be performed.

### Effect of the Invention

The use of the medium for culture of the present invention containing human serum albumin (rHSA) having constant quality and obtained by gene manipulation has led to the effects of retention of cell proliferation capability and production of physiologically active substance, which are of the same level as achieved when a serum-containing medium and/or a conventional serum-free medium containing plasma-derived HSA are/is used, as well as stable quality of the medium and ensured reproducibility of the culture.

This application is based on application No. 210004/1996 filed in Japan, the content of which is incorporated hereinto by reference.

## Claims

1. A medium for culture, containing a human serum albumin obtained by gene manipulation.

2. The medium for culture of claim 1, comprising, as a basal medium, DMEM medium, F12 medium or RPMI1640 medium.

3. The medium for culture of claim 1, wherein the human serum albumin obtained by gene manipulation is the albumin produced by a human serum albumin-producing yeast.

4. The medium for culture of claim 1, further containing insulin.

5. The medium for culture of claim 1, further containing peptone.

6. The medium for culture of claim 1, further containing transferrin.

7. The medium for culture of claim 1, further containing insulin, peptone and transferrin.

8. A method for culturing an animal cell, comprising culturing the animal cell in the medium of claim 1.

9. The method of claim 8, wherein the animal cell itself is capable of producing a physiologically active substance or producing a heterologous physiologically active substance by transformation by genetic engineering.

10. The method of claim 8, wherein the animal cell is a member selected from the group consisting of human kidney cell line, hybridoma, leukocyte, Namalwa cell, BALL-1 cell, fibroblast, lymphocyte, human kidney cell, melanoma cell, Vero cell, Hela cell, CHO cell, WI 38 cell, BHK cell, COS-7 cell, MDCK cell, C127 cell, HKG cell, mouse myeloma cell, human lymphoblast cell, a parent cell for preparing human-human hybridoma, dhfr deficient cell lines thereof, HGPRT deficient cell lines thereof and ouabain resistant cell lines thereof.

11. A method for producing a physiologically active substance, comprising culturing an animal cell capable of producing the physiologically active substance in the medium of claim 1 to produce the physiologically active substance, and harvesting the physiologically active substance from the culture.

12. The method of claim 11, wherein the animal cell itself is capable of producing a physiologically active substance or producing a heterologous physiologically active substance by transformation by genetic engineering.

13. The method of claim 11, wherein the physiologically active substance is a member selected from the group consisting of prourokinase, urokinase, antithrombin-III, tissue plasminogen activator, hepatitis B virus surface antigen, hepatitis pre S-B virus surface antigen, interferon, colony formation stimulating factor and monoclonal antibody.
